# EUROPEAN PATENT SPECIFICATION

(11) **EP 3 982 874 B1**
(45) Date of publication and mention of the grant of the patent: **09.08.2023**
(21) Application number: 20751857.2
(22) Date of filing: 11.06.2020
(51) Int. Cl.: A61C 19/06

(54) **KIT FOR APPLYING EMULSION COMPOSITIONS**
KIT ZUM AUFTRAGEN VON EMULSIONSZUSAMMENSETZUNGEN
KIT D'APPLICATION DE COMPOSITIONS D'ÉMULSION

(30) Priority: 14.06.2019 US 201962861362 P; 06.01.2020 US 202062957366 P
(43) Date of publication of application: 20.04.2022
(73) Proprietor: The Procter & Gamble Company, Cincinnati, OH 45202 (US)
(72) Inventor: CURTIS, Michael, David, Cincinnati, Ohio 45202 (US); HASSE, Jennifer, Marie, Cincinnati, Ohio 45202 (US); RAMJI, Niranjan, Cincinnati, Ohio 45202 (US); SAGEL, Paul, Albert, Cincinnati, Ohio 45202 (US); WASHINGTON, Joe, Leon, III, Cincinnati, Ohio 45202 (US); WILLIS, Laura, Ashley, Cincinnati, Ohio 45202 (US)
(74) Representative: P&G Patent Germany
(86) International application number: PCT/US2020/070137
(87) International publication number: WO 2020/252504

(56) References cited:
- EP-A1- 0 463 979
- WO-A1-2017/182239
- WO-A2-2013/050432
- US-A- 5 240 415
- US-A1- 2009 196 677
- US-B2- 9 072 570

## Description

### FIELD OF THE INVENTION

The present invention is directed to kits for the application of emulsion compositions, such as water-in-oil emulsion compositions, to oral cavity surfaces, such as teeth or gums. The present invention is also directed to devices for the application of hydrophobic compositions to hydrophilic surfaces and/or the application of hydrophilic compositions to hydrophobic surfaces. The present invention is also directed to containers for the storage and dispensing of emulsion compositions, such as water-in-oil emulsion compositions.

### BACKGROUND OF THE INVENTION

Active agents have been delivered to the oral cavity using many devices, such as strips, trays, and the like. However, it can be challenging to apply emulsion compositions to many oral cavity surfaces, such as teeth and/or gums. For example, it can be difficult to apply hydrophobic emulsions directly to the hydrophilic surface of wetted teeth due to energetically unfavorable hydrophobic-hydrophilic interactions. Unfortunately, many applicators can lead to low transfer and to streaking and/or inconsistent application.

Some other strategies to deliver emulsion compositions can include trays, strips, or brushes. While dental trays or brushes can be used to apply hydrophobic emulsions, they can be difficult to clean using soap and/or water after use. Strips can be used to deliver emulsion compositions, but are discarded after each use. Additionally, strips can shift in the oral cavity after initial placement and during the application. As such, there is a need for a that can deliver an emulsion composition with consistent deposition to oral care surfaces.

EP 0 463 979 A1 discloses a kit for applying an oral care composition to an oral cavity surface comprising: (a) an applicator device, the applicator device comprising an applicator handle suitable for self-application and an applicator tip comprising a plurality of internal ridges, wherein the plurality of internal ridges has a second height and extends in a direction at least substantially parallel or parallel to the longitudinal axis of the applicator device, and a plurality of internal channels between the plurality of internal ridges; and (b) a container for the oral care composition, the container comprising an outlet, and a storage portion.

WO 2017/182239 A1 discloses an applicator device, the applicator device comprising an applicator handle suitable for self-application and an applicator tip comprising: (i) at least one external ridge, wherein the at least one external ridge has a first height and lies along; and (ii) a plurality of internal ridges, wherein the plurality of internal ridges has a height and extends in a direction at least substantially parallel to the longitudinal axis of the applicator device; wherein the first height is greater than the second height: and (iii) a plurality of internal channels between the plurality of internal ridges.

### SUMMARY OF THE INVENTION

Disclosed herein is a kit for applying an oral care composition to an oral cavity surface. The kit of the claimed invention is defined in the appended claims.

### BRIEF DESCRIPTION OF THE DRAWINGS

FIG. 1A is a top view of an applicator device.
FIG. 1B is a perspective view of the applicator device of FIG. 1A.
FIG. 2A is a top view of an applicator device.
FIG. 2B is a cross sectional view of the applicator device of FIG. 2A.
FIG. 2C is a magnified view of a portion of the cross-sectional view of FIG. 2B
FIG. 2D is an alternative cross-sectional view of the applicator tip of the applicator device of FIG. 2A.
FIG. 2E is a magnified view of a portion of the cross-sectional view of FIG. 2D
FIG. 2F is a cross sectional view of the applicator device of FIG. 2A.
FIG. 3A is a top view of a comparative applicator device.
FIG. 3B is a perspective view of the comparative applicator device of FIG. 3A.
FIG. 4 is a perspective view of a dispensing nozzle for emulsion compositions.
FIG. 5 is a top view of a dispensing nozzle for emulsion compositions.
FIG. 6 is a perspective view of a comparative dispensing nozzle.
FIG. 7 is a comparison of the % composition transferred and the evenness of application of a variety of applicator devices.
FIG. 8 is a comparison of the % composition transferred between the applicator device of FIG. 2A combined with the nozzle of FIG. 4 (right) and the applicator device of FIG. 3A combined with the nozzle of FIG. 6 (left).
FIG. 9 is a comparison of the evenness of application between the applicator device of FIG. 2A combined with the nozzle of FIG. 4 (right) and the applicator device of FIG. 3A combined with the nozzle of FIG. 6 (left).
FIG. 10 is the % composition transferred and the evenness of transfer between the applicator device of FIG. 2A combined with the nozzle of FIG. 4 (right) and the applicator device of FIG. 3A combined with the nozzle of FIG. 6 (left).
FIG. 11 is a photo of the applicator device of FIG. 2A dispensed from a container with a ribbon orifice the before (left) and after (center) application to a polystyrene surface wetted with saliva. The resulting emulsion composition on the polystyrene surface is picture on the right.
FIG. 12 is a photo of the applicator device of FIG. 3A dispensed from a container with a ribbon orifice the before (left) and after (right) application to a polystyrene surface wetted with saliva.
The resulting emulsion composition on the polystyrene surface is picture on the right.
FIG. 13A is a top view of an applicator device.
FIG. 13B is a cross sectional view of the applicator device of FIG. 13A.

### DETAILED DESCRIPTION OF THE INVENTION

The present invention is directed to kits for the application of emulsion compositions to oral cavity surfaces, such as teeth or gums. The present invention is directed to kits capable of delivering a hydrophobic composition to a hydrophilic surface.

For example, the emulsion compositions disclosed in U.S. Patent App. No. 2018/0133119 and U.S. Patent App. No. 2018/0133121, for their description of the multi-phase oral compositions or emulsion compositions, can be challenging to apply to the hydrophilic surface of wetted teeth. The emulsion compositions disclosed in U.S. Patent App. No. 2018/0133119 and U.S. Patent App. No. 2018/0133121 can be water-in-oil emulsions, with a high proportion of hydrophobic (or "oil" phase) and/or an external hydrophobic phase, which can make application to a hydrophilic surface challenging.

Additionally, water-in-oil emulsion compositions can be difficult to apply with a traditional toothbrush. While, the bristles of a toothbrush can lead to a consistent deposition of the water-in-oil emulsion composition, the water-in-oil emulsion can be difficult to remove from between the bristles of a toothbrush. Over time, this can lead to an accumulation of unapplied material and can lead to the growth of bacterial or other microbes. Thus, the present invention is directed to devices and methods to effectively apply water-in-oil emulsions to hydrophilic surfaces while maintaining the ability to easily clean the device after each use.

One of the advantages of applying a water-in-oil emulsion with a traditional toothbrush is the bristles can provide a mechanism to reach all portions of the surface of teeth as individual bristles can bend and separate to spread the water-in-oil emulsion compositions across the surfaces of teeth similar to how a trowel can spread mortar consistently. However, the water-in-oil emulsion also can deposit between individual bristles, which can be difficult to remove by washing with water due to the high hydrophobic character of the water-in-oil emulsions. Thus, the present invention is directed to an applicator that can spread the water-in-oil emulsion across the surface of teeth, while still maintaining the ability to effectively clean the applicator tip for repeat usage.

As described herein, pairing the disclosed applicator with an appropriate container leads to an improved application. The applicator device can be designed with an internal cavity to hold the emulsion composition prior to application. Thus, the container can have a nozzle that is designed to optimally fill the internal cavity of the applicator device. If the consumer optimally fills the internal cavity, there can be less spillage, and a more consistent deposition from the applicator device during application.

### Definitions

By "oral care composition", as used herein, is meant a product, which in the ordinary course of usage, is not intentionally swallowed for purposes of systemic administration of particular therapeutic agents, but is rather retained in the oral cavity for a time sufficient to contact dental surfaces or oral tissues. Examples of oral care compositions include dentifrice, tooth gel, subgingival gel, mouth rinse, mousse, foam, mouth spray, lozenge, chewable tablet, chewing gum, tooth whitening strips, floss and floss coatings, breath freshening dissolvable strips, or denture care or adhesive product. The oral care composition may also be incorporated onto strips or films for direct application or attachment to oral surfaces.

The term "dentifrice", as used herein, includes tooth or subgingival -paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

The term "phase" as used herein means a physically distinct region or regions, which may be continuous or discontinuous, having one or more properties that are different from another phase. Non-limiting examples of properties that may be different between phases include composition, viscosity, solubility, hydrophobicity, hydrophilicity, and miscibility.

The term "multi-phase oral composition" as used herein comprises a mixture of two or more phases that are immiscible with each other, for example such as water in oil emulsions. The phases may be continuous, discontinuous, or combinations thereof. Examples of multi-phase oral compositions include emulsions, such as water in oil emulsions. Examples of multi-phase oral compositions also include oil-in-water emulsions, water-in-oil-in-water emulsions, and oil-in-water-in-oil emulsions. Examples of multi-phase oral compositions also include compositions where the phases are multi-continuous including bi-continuous, layered, striped, marbled, ribbons, swirled, and combinations thereof.

The term "emulsion" as understood herein is an example of a multi-phase composition wherein: 1) at least one of the phases is discontinuous and 2) at least one of the phases is continuous. Examples of emulsions include droplets of water dispersed in oil. In this example the water and oil would be mutually immiscible with each other, water would be the discontinuous phase, and the oil would be the continuous phase.

The term "water-in-oil emulsion" as understood herein is an example of an emulsion wherein 1) the discontinuous phase is aqueous, and 2) the continuous phase is hydrophobic.

The term "aqueous phase" as understood herein is at least one phase that comprises water and an active agent, and is immiscible with the hydrophobic phase. In certain embodiments, each part of the aqueous phase contains at least 2% of the active agent by weight of the aqueous phase. Optionally the aqueous phase may further comprise ingredients that are water soluble, water miscible, or combinations thereof, such as for example water soluble solvents, alcohol, polyethylene glycol, carbopol, etc. or mixtures thereof. In some embodiments, if and when immiscible fillers are added to the aqueous phase, the percentage of the aqueous phase in the composition is calculated by excluding the immiscible filler.

The term "hydrophobic phase" as understood herein means all components of the composition that are immiscible with the aqueous phase. In certain embodiments the hydrophobic phase may further comprise ingredients that are soluble, miscible or combinations thereof in the hydrophobic phase, such as for example hydrocarbon solvents dissolved into the hydrophobic phase, polyethylene dissolved into the hydrophobic phase, microcrystalline wax dissolved into the hydrophobic phase, or mixtures thereof.

By "a sufficient period of time to achieve a benefit," as used herein is meant that the composition is used or worn by the participant or the participant is instructed to use or wear the composition for greater than about 10 seconds; or greater than about 1 minute, such as from about 2.5 minutes to about 12 hours (for example overnight treatment), or from about 3 minutes to about 180 minutes; or greater than about 5 minutes, such as from about 5 minutes to about 60 minutes; or greater than about 10 minutes, such as from about 10 minutes to about 60 minutes; or from about 1, 5, 10, or 15 minutes to about 20, 30, 60, 120 minutes per application; or any other numerical range, which is narrower and which falls within such broader numerical range, as if such narrower numerical ranges were all expressly written herein. In addition, the treatments may be applied from about 1, 2, or 3 times a day to about 4, 5, 6 or 7 times a day. The treatments may be applied for from about 1, 2, 3, 4, 5, 6, or about 7 days to about 8, 9, 10, 11, 12, 13, 14, 21, or 28 days or any other numerical range, which is narrower and which falls within such broader numerical range, as if such narrower numerical ranges were all expressly written herein. In some embodiments the wear time is not the same across different days the treatment is applied. Further, the length of treatment to achieve the desired benefit, for example, tooth whitening, may last for a specified period of time, which may be repeated if necessary, for example from about one day to about six months, in particular from about one day to about 28 days, or from about 7 to about 28 days. The optimal duration and frequency of application will depend on the desired effect, the severity of any condition being treated, the health and age of the user and like considerations.

"Elastomer," as used herein, is defined as a polymer with rubber-like elasticity. An elastomer is a polymer with viscoelasticity, weak intermolecular forces, low Young's modulus, and high failure strain compared with other polymers and materials.

The term "ridge" is used to mean a narrow, raised band of material. A ridge has a long axis and a short axis or height.

The term "about" means that amounts, sizes, formulations, parameters, and other quantities and characteristics are not and need not be exact, but can be approximate and/or larger or smaller, as desired, reflecting tolerances, conversion factors, rounding off, measurement errors, and the like, and other factors known to those of skill in the art. In general, an amount, size, formulation, parameter or other quantity or characteristic is "about" or "approximate" whether or not expressly stated to be such. The term "about" also encompasses amounts that differ due to different equilibrium conditions for a composition resulting from a particular initial mixture. Whether or not modified by the term "about," the claims include equivalents to the quantities. The term "about" can mean within 10% of the reported numerical value, preferably within 5% of the reported numerical value.

Several types of ranges are disclosed in the present invention. When a range of any type is disclosed or claimed, the intent is to disclose or claim individually each possible number that such a range could reasonably encompass, including end points of the range as well as any sub-ranges and combinations of sub-ranges encompassed therein.

The foregoing summary is not intended to define every aspect of the invention, and additional aspects are described in other sections. In addition, the invention includes, as an additional aspect, all embodiments of the invention narrower in scope in any way than the variations defined by specific paragraphs set forth herein. For example, certain aspects of the invention that are described as a genus, and it should be understood that every member of a genus is, individually, an aspect of the invention. Also, aspects described as a genus or selecting a member of a genus should be understood to embrace combinations of two or more members of the genus. With respect to aspects of the invention described or claimed with "a" or "an," it should be understood that these terms mean "one or more" unless context unambiguously requires a more restricted meaning. The term "or" should be understood to encompass items in the alternative or together, unless context unambiguously requires otherwise. If aspects of the invention are described as "comprising" a feature, embodiments also are contemplated "consisting of" or "consisting essentially of" the feature.

Features of the compositions and methods are described below. Section headings are for convenience of reading and not intended to be limiting per se. The entire document is intended to be related as a unified disclosure, and it should be understood that all combinations of features described herein are contemplated, even if the combination of features are not found together in the same sentence, or paragraph, or section of this document. It will be understood that any feature of the methods or compounds described herein can be deleted, combined with, or substituted for, in whole or part, any other feature described herein.

### Emulsion Compositions

The applicator device, as described herein, is specially adapted for the application of an emulsion composition to an oral cavity surface. The emulsion composition can be an oil-in-water emulsion composition, a water-in-oil emulsion composition, and/or combinations thereof. Emulsion compositions can be applied to the surface of teeth, gums, cheeks, tongue, or any other oral cavity surface.

One example of an emulsion composition that can be deposited to the oral cavity are the water-in-oil emulsion compositions disclosed by U.S. Patent App. No. 2018/0133119 and U.S. Patent App. No. 2018/0133121, reference for their description of the multi-phase oral compositions or emulsion compositions. The water-in-oil emulsion compositions can have an active agent in the aqueous phase or the hydrophobic phase. Suitable active agents include those described in U.S. Patent App. No. 2018/0133119 and U.S. Patent App. No. 2018/0133121. Suitable active agents include bleaching agents, one or more anticalculus agent(s), a fluoride ion source, antimicrobial agent(s), dentinal desensitizing agent(s), anesthetic agent(s), antifungal agent(s), antiinflammatory agent(s), selective H-2 antagonist(s), anticaries agent(s), nutrient(s), erythritol, probiotics, and mixtures thereof. Specific examples of active agents include, but are not limited to, hydrogen peroxide, fluoride salts, stannous salts, zinc salts, oxalates, cetylpyridinium chloride and mixtures thereof. One of the purposes of the water-in-oil emulsion compositions can be to deliver an active agent for a sufficient period of time to achieve a benefit of the active agent. For example, if the active agent is hydrogen peroxide, the composition needs to contact the surface of teeth for a sufficient period of time to whiten the teeth. If the active agent is stannous fluoride, the composition needs to contact the surface of teeth and/or gums for a sufficient period of time to deposit stannous ions and/or fluoride ions to provide an antigingivitis, anti sensitivity, and/or anticaries benefit.

The emulsion composition can have a cone penetration value of from about 100 to about 300, preferably in the range from about 150 to about 250, and more preferably in the range of from about 170 to about 200 or any other numerical range, which is narrower and which falls within such broader numerical range, as if such narrower numerical ranges were all expressly written herein, as measured according to ASTM method D937-07. Without being bound by theory, the cone penetration consistency value of the water-in-oil emulsion composition may be a factor to ensure that the water-in-oil emulsion composition: 1) is substantive and does not run down the teeth or run out of the delivery carrier during application or during use; and 2) releases an effective amount of the bleaching agent or active agent during use. Specifically, if the cone penetration consistency value of the water-in-oil emulsion composition is too high, the water-in-oil emulsion composition may not be substantive and run down the teeth or run out of the delivery carrier during application or during use. In contrast, if the cone penetration consistency value of the water-in-oil emulsion composition is too low, the water-in-oil emulsion composition may not release an effective amount of the bleaching agent or active agent during use.

Teeth wetted with saliva present a unique challenge for the deposition of water-in-oil emulsion compositions. Wetted teeth are a hydrophilic surface. Many water-in-oil emulsion compositions have a high hydrophobic character and/or the hydrophobic phase as the external phase. Thus, it can be challenging to deposit a hydrophobic composition onto a hydrophilic surface.

### Other Compositions

While specific reference is made herein to the application of emulsion compositions, hydrophobic compositions, and/or water-in-oil emulsion compositions, the applicator devices described herein can also be used to apply a variety of other compositions, such as the jammed emulsions of U.S. Provisional Application No. 62/838,350, the compositions ofPCT/CN2019/091272, the compositions of U.S. Provisional Application No. 62/834,625, or any other oral care composition that can be applied directly to an oral cavity surface.

### Applicator Device (10)

The applicator device (10) described herein, can be designed to spread the emulsion composition across oral cavity surfaces. Additionally, the applicator device (10) described herein, can be designed to contain the emulsion composition and facilitate the transfer of the emulsion composition by preventing the material from spilling during the application. Moreover, the applicator device (10) described herein, can be made from a material that can facilitate the transfer of the emulsion composition to the targeted oral cavity surface and have properties to promote comfort to users during application. As such, the applicator device (10) can comprise an applicator handle (20) suitable for self-application and an applicator tip (30) at least one of (a) at least one external ridge; (b) a plurality of internal ridges; and/or (c) a plurality of internal channels between the plurality of internal ridges.

### Applicator Handle (20)

The applicator handle (20) can be sized for use in self-application or sized for application by dental professional. The applicator handle (20) can be straight or substantially straight. The applicator handle (20) can also be curved, angled, or bendable to facilitate application to hard to reach oral care surfaces, such as tooth surfaces in the back of the oral cavity.

The applicator handle (20) can be made from any suitable material, such as for example, a polymer, a polymer alloy, an elastomer, a metal, a metal alloy, glass, and/or combinations thereof. The applicator handle (20) can have gripping elements or aesthetic elements. The applicator handle (20) can be opaque, translucent, transparent, and/or combinations thereof.

Suitable polymer materials include, but are not limited to, polypropylene, polyethylene, polyethylene terephthalate, and/or combinations thereof.

The applicator handle (20) can have a shore durometer rating of from about 70 D to about 90 D, from 75 D to about 85 D, from about 77 D to about 83 D, from about 45 A to about 75 A, from about 50 A to about 80 A, from about 50 A to about 70 A, or from about 60 A to about 80 A. The applicator handle (20) can have a shore durometer rating that is greater than the shore durometer rating of the applicator tip (30) so that the applicator tip (30) will be flexible within the oral cavity. The durometer rating can be determined by ASTM Test No. D2240, which is herein incorporated by reference.

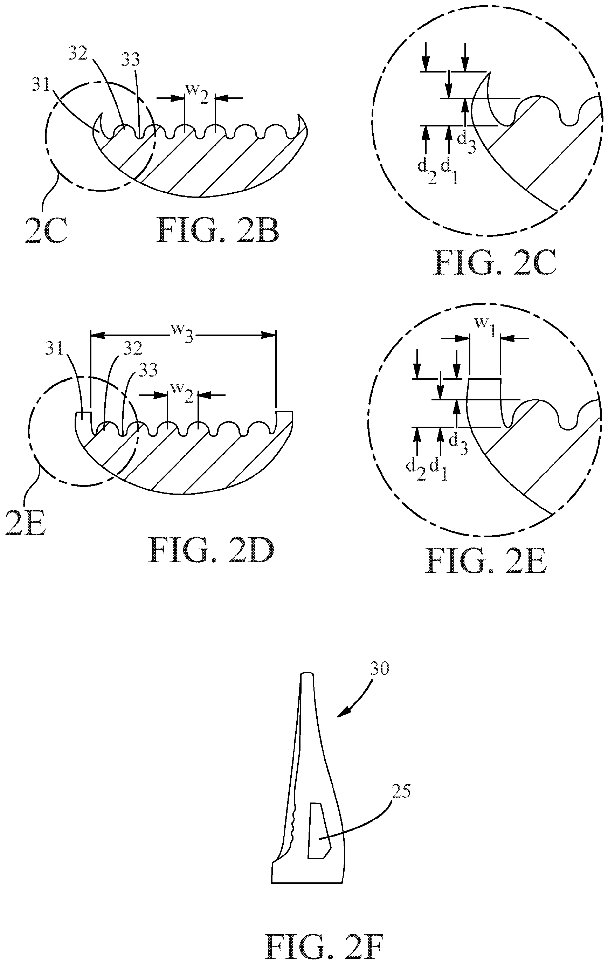

As in FIG. 2F, the applicator handle (20) can have an applicator insert (25) that can fit within a void in the applicator tip (30) to assist in the connection between the tip (30) and the handle (20) and/or provide a platform to resist the flexibility of the applicator tip (30), which can increase application precision, accuracy, and/or comfort.

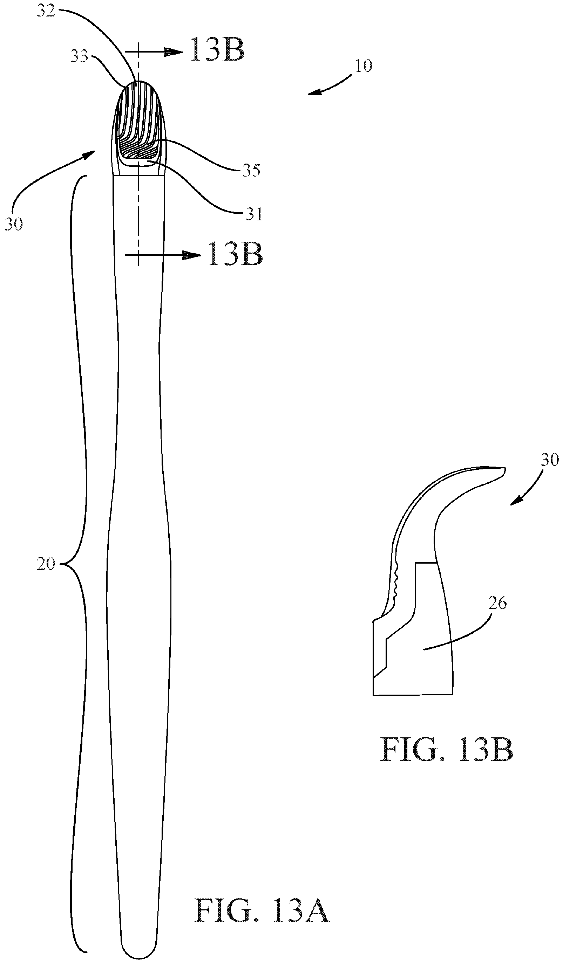

As in FIG. 13B, the applicator handle (20) can have an applicator posterior support (26) that can assist in the connection between the tip (30) and the handle (20) and/or provide a platform to resist the flexibility of the applicator tip (30), which can increase application precision, accuracy, and/or comfort.

The applicator tip (30) can be reversibly attached to the applicator handle (20) using any suitable means, such as the connector disclosed in U.S. Patent Application Publication No. 2019/0246780.

### Applicator Tip (30)

As described herein, the applicator tip (30) can be designed to facilitate transfer of an emulsion composition from the applicator tip to an oral care surface. For example, the applicator tip (30) can be specifically designed to deliver a substantially hydrophobic composition to a hydrophilic surface, such as wetted teeth.

The applicator tip (30) can be made from a material that is suitable for application to oral care surfaces. For example, the applicator tip (30) can be made from food & drug grade materials, materials on the GRAS (Generally Regarded As Safe) list, or other applicable materials approved for use within the oral cavity according to local laws.

The applicator tip (30) can be made from any suitable material, such as for example, a polymer, a polymer alloy, an elastomer, a metal, a metal alloy, glass, and/or combinations thereof.

Suitable polymer materials include, but are not limited to, polypropylene, polyethylene, polyethylene terephthalate, and/or combinations thereof.

Suitable elastomer materials include, but are not limited to, thermoplastic elastomers, a styrenic, a copolyester, a polyurethane, a polyamide, a polyolefin blend, a polyolefin alloy, a reactor TPO, a polyolefin plastomer, a polyolefin elastomer, and/or combinations thereof. Suitable elastomers include, for example, an elastomer made from PolyOne^{®} under the Versaflex^{™} or Dynaflex^{™} product lines, from Hapco, Inc under the Steralloy^{™} product line, or from United Soft Plastics under the Unisoft Standard SEBS-based thermoplastic elastomer.

The applicator tip (30) can have a shore durometer rating of from about 25 A to about 90 A, from about 40 A to about 85 A, from about 45 A to about 75 A, or from about 60 A to about 70 A.

The applicator tip (30) for the applicator device may have a longitudinal axis/extension being defined as the axis/extension between a proximal end and a distal end of the applicator tip (30). As described herein, the term "proximal end" means the end of the applicator tip (30) which may be attached or attachable to the applicator handle (20) of the applicator device (10), whereas the term "distal end" means the end of the applicator tip (30) being opposite the proximal end, i.e. being furthest away from the applicator handle (20)/at the loose/free end of the applicator tip (30). A longitudinal application direction is defined by an applicator movement in the direction towards the distal end or towards the proximal end of the applicator tip (30), i.e. along the longitudinal extension of the applicator tip (30).

The applicator tip (30) can extend from the applicator handle (20) and may be either repeatedly attachable to and detachable from the applicator handle (20), or the applicator tip (30) may be non-detachably connected to the applicator handle (20).

The applicator tip (30), as described herein, comprises at least one external ridge (31). The at least one external ridge (31) can lie along at least a majority of the perimeter of the applicator tip (30) such that the at least one external ridge (31) forms an internal cavity (35) for the emulsion composition to be placed in.

The height of the at least one external ridge (31) can be greater at the proximal end of the applicator tip (30) and be smaller at the distal end of the applicator tip (30). The at least one external ridge (31) can be continuous and surround the entire perimeter of the applicator tip (30). The at least one external ridge (31) can be a continuous or discontinuous ridge surrounding from about 25% to about 95%, from about 33% to about 90%, or from about 40% to about 80% of the perimeter of the applicator tip (30). Leaving a portion of the perimeter of the applicator tip (30) free from an external raised member, such as the at least on external ridge (31) can allow for the emulsion composition to be accurately and efficiently transferred from the applicator tip (30) to the oral cavity surface without trapping emulsion composition within the internal cavity (35). The free portion of the perimeter can allow the emulsion composition to flow out of the applicator tip. The at least one external ridge (31) can extend in an equal distances in either direction from the distal end of the applicator tip (30).

The applicator tip (30), as described herein, comprises a plurality of internal ridges (32). The plurality of internal ridges (32) extend in a direction at least substantially parallel or parallel to the longitudinal axis of the applicator device (10). In contrast, the bristles of a toothbrush are at least substantially perpendicular or perpendicular to the longitudinal axis of the toothbrush.

At least a portion of the plurality of internal ridges (32) can be straight or at least substantially straight. At least a portion of the plurality of internal ridges (32) can be curved. At least a portion of the plurality of internal ridges (32) can be a combination of straight or at least substantially straight portions and curved portions. The plurality of internal ridges (32) can be equally spaced or unequally spaced across the surface of the applicator.

At least a portion of the plurality of internal ridges (32) can terminate at the superior edge or within about 1 mm, within about 5 mm, or within about 10 mm of the superior edge of the applicator tip (30). All of the plurality of internal ridges (32) can terminate at the superior edge or within about 1 mm, within about 5 mm, or within about 10 mm of the superior edge of the applicator tip (30).

The applicator tip (30), as described herein, comprises a plurality of internal channels (33) that are between the plurality of internal ridges (32). The width of the internal channels (33) can be identical or vary in size. The plurality of internal ridges (32) and plurality of internal channels (33) facilitate the transfer and spreading of an emulsion composition to an oral cavity surface similar to a trowel with mortar. The plurality of internal channels (33) can allow for emulsion composition to evenly spread along the surface of wetted teeth. The plurality of internal ridges (32) and plurality of internal channels (33) can be easier to remove any remaining emulsion composition after application because the plurality of ridges (32) are fixed in location, unlike toothbrush bristles, and can not stick together. Thus, the emulsion composition can be more easily wiped or rinsed away.

The plurality of internal ridges (32) can comprise from 2 to about 20, from about 5 to about 15, or from about 3 to about 25 of internal ridges. The plurality of internal channels (33) can comprise from 2 to about 20, from about 5 to about 15, or from about 3 to about 25 of internal channels. The at least one external ridge (31) can comprise 1, 2, 3, or more external ridges that surround at least a majority of the perimeter of the applicator tip (30).

The plurality of internal ridges (32) can be smaller in height than the at least one external ridge (31). The difference in height between the plurality of internal ridges (32) and the at least one external ridge (31) can form the internal cavity (35) of the applicator tip (30) for the placement of the emulsion composition.

The height of each of the plurality of the internal ridges (32) can be identical or they can vary along the surface of the applicator tip (30). The volume of each of the plurality of internal channels (33) can be identical or they can vary along the surface of the applicator tip (30). For example, the volume of the plurality of internal channels (33) can be greater towards the middle of the applicator tip (30) and less approaching the at least one external ridge (31).

The applicator tip (30) can be clear, transparent, translucent, and/or opaque. The applicator tip (30) can be colored to allow the user to easily see the emulsion composition on the applicator tip. The applicator tip (30) and the emulsion composition can be different colors, contrasting colors, or either the applicator tip (30) or the emulsion composition can be colored so that the applicator tip and the emulsion composition can be easily distinguished by the user during application to an oral cavity surface.

The distance, d₁ in FIG. 2B and 2C, from the valley of the internal channel (34) of the plurality of internal channels (33) to the peak of the plurality of the internal ridges (33) can be from about 0.001 mm to about 1 mm, from about 0.01 mm to about 0.5 mm, or from about 0.02 mm to about 0.4 mm. The distance, d₁ in FIG. 2B and 2C, from the valley of the internal channel (34) of the plurality of internal channels (33) to the peak of the plurality of the internal ridges (33) can be consistent throughout the entire tip or d₁ can vary longitudinally, latitudinally, or from the distal end to the proximal end of the applicator tip (30).

The distance, d₂ in FIG. 2B and 2C, from the valley of the internal channel (34) of the plurality of internal channels (33) to the peak of the at least one external ridge (31) can be from about 0.01 mm to about 1.0 mm, from about 0.1 mm to about 0.9 mm, or from about 0.25mm to about 0.75 mm. The distance, d₂ in FIG. 2C, from the valley of the internal channel (34) of the plurality of internal channels (33) to the peak of the at least one external ridge (31) can be consistent throughout the entire tip (30) or d₂ can vary longitudinally, latitudinally, or from the distal end to the proximal end of the applicator tip (30).

The depth, d₃ in FIG. 2B and 2C, of the internal cavity (35), can be from 0 to about 5 mm, from 0 to about 2.0 mm, up to about 1.5 mm, up to about 1.25 mm, from about 0.01 mm to about 2.0 mm, or from about 0.1 mm to about 2.0 mm. The depth of the internal cavity (35), d₃, can be consistent throughout the applicator tip (30) or d₃ can vary longitudinally, latitudinally, or from the distal end to the proximal end of the applicator tip (30).

The width of the at least one external ridge (31), W₁ in FIG. 2C, can be from about 0.10 mm to about 2.5 mm, from about 0.25 mm to about 2.2 mm, or from about 0.35 mm to about 2.2 mm. The at least one external ridge (31) can be flat with a width (i.e. a plateau) as in FIG. 2C, be a peak as in FIG. 2B, and/or rounded.

The width between two internal ridges of the plurality of internal ridges (32), W₂ in FIG. 2C, can be from about 0.01 to about 2.0 mm, from about 0.10 mm to about 1.5 mm, or from about 0.25 mm to about 1 mm.

The width of the internal cavity (35), W₃ in FIG. 2C, can be from about 1.0 mm to about 10 mm, from about 4.0 mm to about 9.0 mm, or from about 5.0 mm to about 8.0 mm.

The volume of the space between the at least on external ridge (31) along the surface of the applicator tip (30) can be known as the pocket volume of the applicator tip (30). The applicator tip (30) can have a pocket volume of from about 5 mm³ to about 50 mm³, from about 10 mm³ to about 25 mm³, or from about 10 mm³ to about 20 mm³.

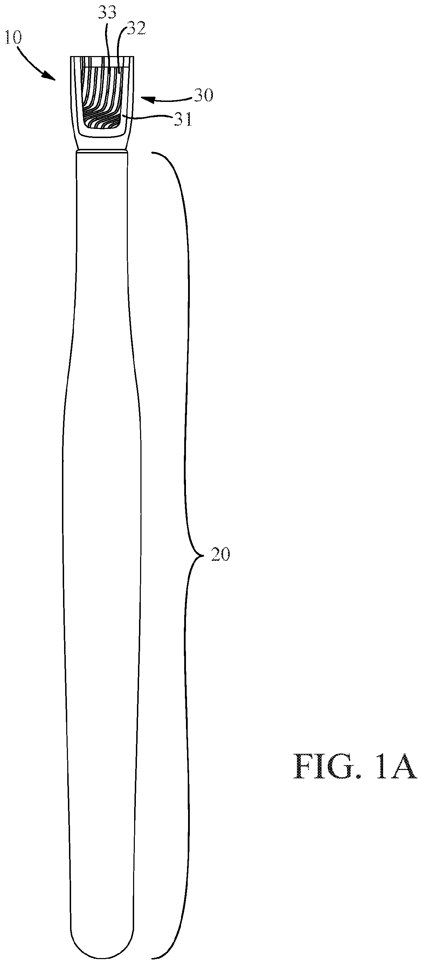

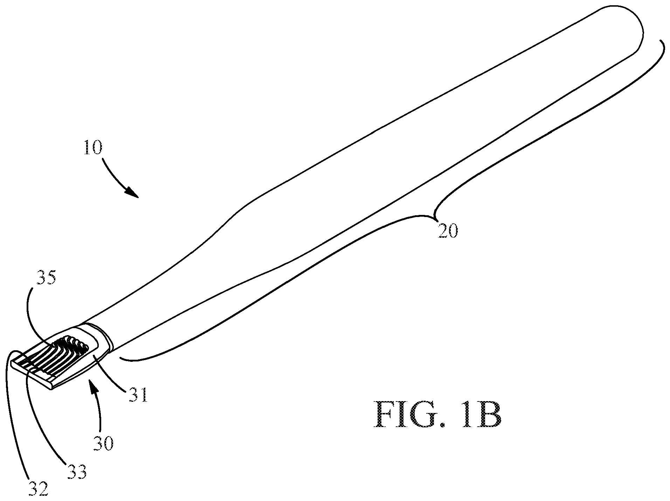

As shown in FIG. 1A and FIG. 1B, the applicator device (10) can have a handle (20) and an applicator tip (30). The applicator tip (30) can have at least one external ridge (31). In one embodiment, as shown in FIG. 1A and 1B, the at least one external ridge (31) comprises a single ridge in a "U" shape running from one end of the distal end of the applicator tip (30) to the opposite end of the distal end of the applicator tip (30). In one embodiment, as shown in FIG. 1A and 1B, the plurality of internal ridges (32) have a straight portion and a curved portion. Additionally, in one embodiment, as shown in FIG. 1A and 1B, the distal end of the applicator tip (30) is straight.

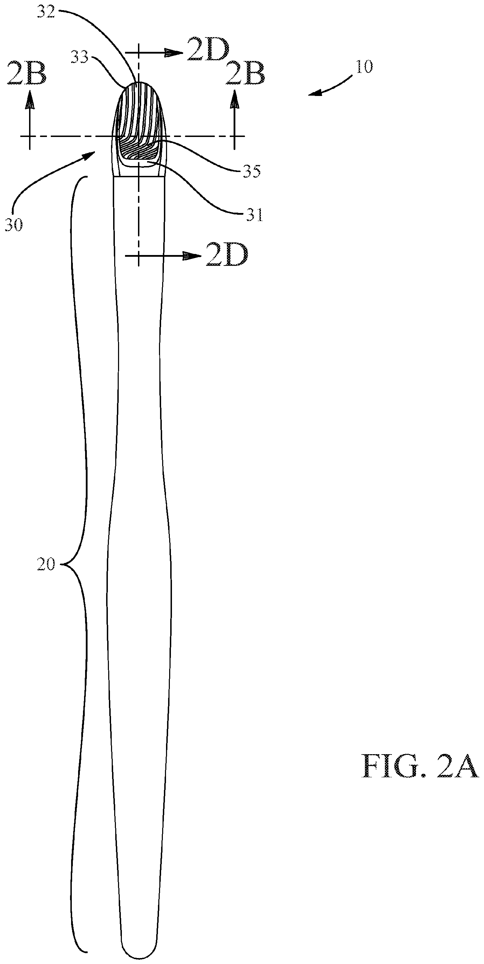

In one embodiment, as shown in FIG. 2A, the distal end of the applicator tip (30) can be curved, which can increase application precision, accuracy, and/or comfort.

FIG. 2B is a cross-sectional view of the applicator tip (30) of FIG. 2A. As shown in FIG. 2C, the applicator tip (30) can comprise at least one external ridge (31), a plurality of internal ridges (32), and a plurality of internal channels (33) between the plurality of internal ridges.

FIG. 2C is an alternative cross-sectional view of the applicator tip (30) of FIG. 2A. As shown in FIG. 2C, the applicator tip (30) can comprise at least one external ridge (31), a plurality of internal ridges (32), and a plurality of internal channels (33) between the plurality of internal ridges.

FIG. 2D is a cross-sectional view of FIG. 2A. In one embodiment, the applicator tip (30) can have an applicator insert (25) to assist in connecting the applicator tip (30) to the applicator handle (20) and/or assist in the application of the emulsion composition.

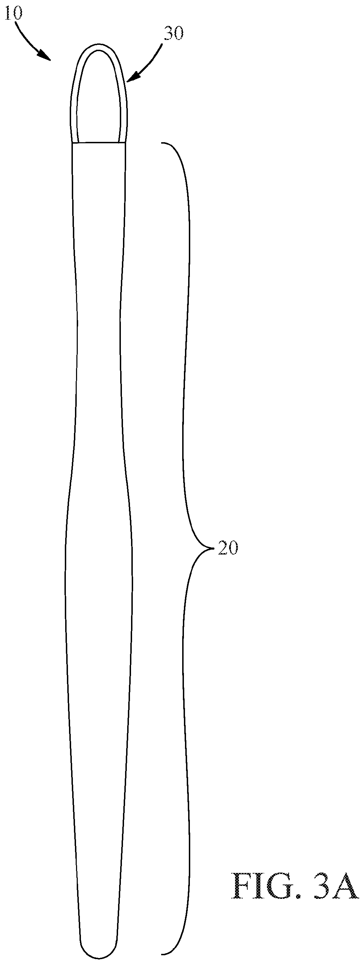

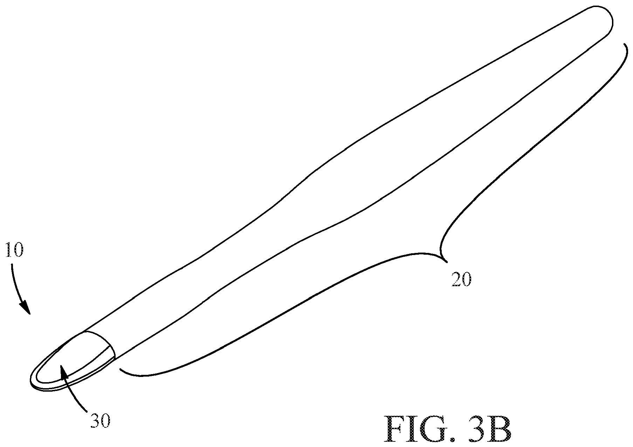

FIG. 3A and FIG. 3B demonstrate a comparative applicator device that do not have a plurality of internal ridges (32) or a plurality of internal channels (33) between the plurality of internal ridges.

FIG. 13A and FIG. 13B demonstrate an applicator device (10) that has an applicator posterior support (26) that can adjust the flexibility of the applicator tip (10).

### Container (50)

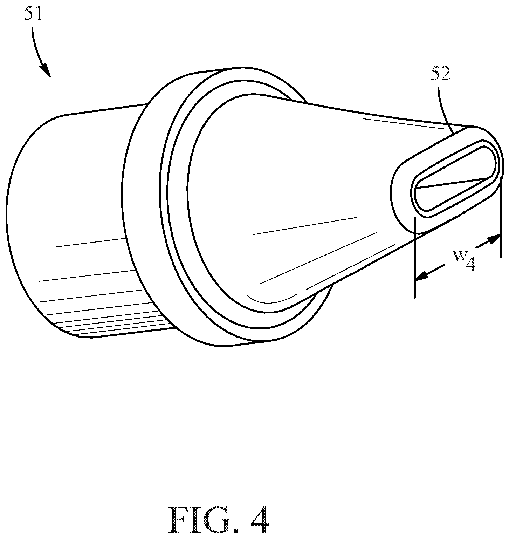

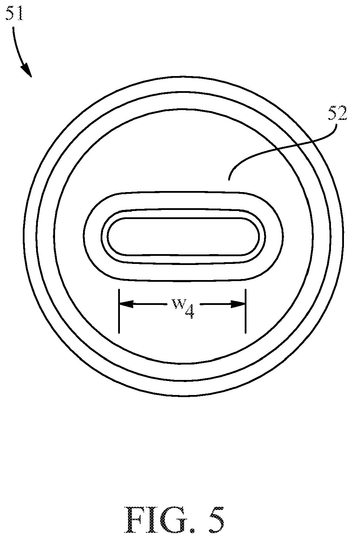

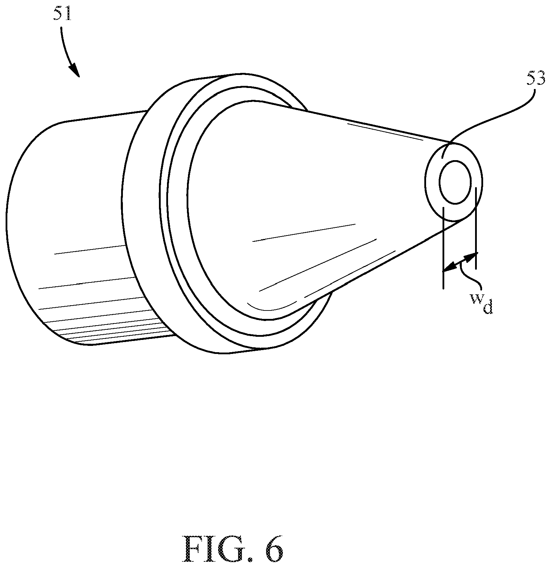

The applicator device (10), as described herein, can be paired within a kit further comprising a container (50). The container (50) can comprise a nozzle (51) with a ribbon orifice (52), as shown in FIG. 4 and 5, or a circular orifice (53), as shown in FIG. 6. The ribbon orifice (52) can be described by the total width of the ribbon orifice (52), W₄, as shown in FIG. 4 and FIG. 5. The circular orifice (53) can be described by the diameter of the circular orifice (53), W_{d}, as shown in FIG. 6. The container (50) can comprise the emulsion composition, as described herein, within a storage portion (54).

As described herein, the applicator device (10) can be specifically designed to apply the emulsion composition to an oral cavity surface, such as teeth. However, application performance can be further increased if the internal cavity (35) of the applicator device is efficiently filled with the emulsion composition. For example, it can be advantageous to eliminate or reduce globular spreading during the application process.

Thus, the nozzle (51) can be designed to efficiently fill the internal cavity (35) of the application device (10). For example, the W₄ of the ribbon orifice (52) can be about the same length or slightly less than the length of W₃, the width of the internal cavity (35) of the applicator device (10). The W₄ of the ribbon orifice (52) can be equal to, from about 5% less than to about 40% less than, from about 5% less than to about 30% less than, or from about 1% less than to about 10% less than the length of W₃. The W₄ can from about 1.0 mm to about 10 mm, from about 4.0 mm to about 9.0 mm, or from about 5.0 mm to about 8.0 mm.

The W_{d} of the circular orifice (53) can be about the same length or slightly less than the length of W₃, the width of the internal cavity (35) of the applicator device (10). The W_{d} of the circular orifice (53) can be equal to, from about 5% less than to about 40% less than, from about 5% less than to about 30% less than, or from about 1% to about 10% less than the length of W₃.

### Delivery Carrier

The emulsion composition can also be applied to a delivery carrier that can be applied to an oral cavity surface as described in U.S. Patent App. No. 2018/0133119 and U.S. Patent App. No. 2018/0133121. For example, the delivery carrier may comprise a first layer of a carrier material and a second layer comprising the emulsion composition described herein, whereby an active agent is releasably located within the present composition. A suitable first layer may comprise a delivery carrier including a strip of material, a dental tray, a sponge material, and mixtures thereof. In certain embodiments, the delivery carrier may be a strip of material, such as a permanently deformable strip. Suitable strips of material or permanently deformable strips are for example disclosed in U.S. Pat. Nos; 6,136,297; 6,096,328; 5,894,017; 5,891,453; and 5,879,691; and in U.S. Pat. Nos. 5,989,569 and 6,045,811; and in patent application US 2014/0178443 A1, for their description of strips.

One example of a strip is a wax strip. A suitable wax strip can be one that deforms at the temperature of the oral cavity. The wax strip can have a softening point of from about 90 °F to about 212 °F, from about 30 °C to about 100 °C, from about 40 °C to about 90 °C, or from about 60 °C to about 85 °C. The softening point can be determined by ASTM D36 using the ring-and-ball apparatus.

The wax strip can be shaped and sized to fit over one or more teeth present in the oral cavity. The wax strip can have a length of from about 20 mm to about 100 mm, from about 40 mm to about 80 mm, or from about 50 mm to about 70 mm. The height of the wax strip can be from about 5 mm to about 50 mm, from about 10 mm to 40 mm, or from about 20 mm to about 35 mm.

The wax strips can have a thickness of from about 5 gauge to about 50 gauge, from about 10 gauge to about 40 gauge, or from about 20 gauge to about 35 gauge. The thickness of the wax strip can be a thickness suitable for application of an oral care composition to the oral cavity. For example, the wax strip can be too thin, which can lead to ripping in the mouth during application. Additionally, the wax strip can be too thick, which can lead to a wax strip that does not soften to allow for the application to uneven surfaces in the oral cavity.

The wax strips can be rolled wax strip. The wax strips can be the wax strips provided by Freeman Manufacturing & Supply Co (Avon, OH).

The delivery carrier may be attached to the teeth via an attachment means that is part of the delivery carrier, for example the delivery carrier may be of sufficient size that, once applied the delivery carrier overlaps with the oral soft tissues rendering more of the teeth surface available for active agent delivery. The delivery carrier may also be attached to the oral cavity by physical interference or mechanical inter-locking between the delivery carrier and the oral surfaces including the teeth.

The delivery carrier maybe transparent or translucent to electromagnetic radiation with wavelengths from about 200nm to about 1700nm. In certain embodiments, the delivery carrier allows from about 10%, 20%, or 30 % to about 40%, 50%, 60%, 70%, 80%, 90%, or 100% of electromagnetic radiation from about 400 nm to about 500 nm to pass through.

Where the delivery carrier is a strip of material, the second layer composition may be coated on the strip, or be applied by the user to the strip, or be applied by the user to the teeth and then the strip may be placed over the coated teeth. The amount of composition applied to the strip or teeth may depend upon the size and capacity of the strip, concentration of the active and the desired benefit; for example from about 0.0001, 0.001 or 0.01 grams to about 0.01, 0.1, 1, or 5 grams may be used or any other numerical range, which is narrower and which falls within such broader numerical range, as if such narrower numerical ranges were all expressly written herein, of composition, in particular from about 0.001g to about 0.5g or from about 0.1g to about 0.4g of multi-phase oral composition may be used. In addition, from about 0.0001, 0.001 or 0.01 grams to about 0.01, 0.1, 0.5, or 1 grams composition per square centimeter of material (g/cm²) may be used or any other numerical range, which is narrower and which falls within such broader numerical range, as if such narrower numerical ranges were all expressly written herein; in certain embodiments less than about 0.2 g/cm², from about 0.0001g/cm² to about 0.1 g/cm², or from about 0.01 g/cm² to about 0.04 g/cm². In addition or alternatively, from about 1 microgram to about 5000 micrograms active agent per square centimeter of material (microgram/cm²), preferably from about 10 micrograms/cm² to about 500 micrograms/cm², and more preferably from about 50 micrograms/cm² to about 100 micrograms/cm² active agent per square centimeter of material may be used.

The delivery carriers can also be combined within a kit with the container (50) as described herein. A container (50) with a ribbon orifice (52) in the nozzle (51) can be advantageous for efficiently loading the emulsion composition. The ribbon orifice (52) can be sized to match the desired width of the emulsion composition based on the delivery carrier selected. For example, the ribbon orifice (52) can be sized to match the size of teeth such that the bead of emulsion composition extruded onto a strip will be the correct width to cover substantially all of the tooth's surface. Additionally, the ribbon orifice (52) can be sized to match the size of the width of the internal cavity (35).

### All-in-one Applicator

The emulsion composition can also be delivered from within an applicator, an all-in-one applicator. The all-in-one applicator can have (i) an internal chamber that store the emulsion composition or oral care composition, (ii) an applicator tip, the applicator tip comprising (a) two or more orifices, (b) an internal cavity to store the composition prior to application, (c) at least one external ridges, (d) a plurality of internal ridges, and (e) a plurality of internal channels between the plurality of internal ridges.

The all-in-one applicator can have two or more orifices, three or more, or four or more orifices which can help to evenly spread the emulsion compositions on the applicator tip when the emulsion composition is forced out of the internal chamber, such as by squeezing the all-in-one applicator. The orifices of the all-in-one applicator can be circular, ribbon, oval, and/or combinations thereof.

### EXAMPLES

The invention is further illustrated by the following examples, which are not to be construed in any way as imposing limitations to the scope of this invention. Various other aspects, modifications, and equivalents thereof which, after reading the description herein, may suggest themselves to one of ordinary skill in the art without departing from the spirit of the present invention or the scope of the appended claims.

Applicator devices and containers were screened for the % of emulsion transferred and evenness of the application of an emulsion composition to a tooth surface. Applicator devices were tested using *in vivo* and *in vitro* models. A blue dye was added to the emulsion composition to facilitate visual and quantitative analysis of the various applicator kits.

In the *in vivo* model, 0.05 to 0.15 g of the emulsion composition was loaded onto various applicator devices from a container with a nozzle having either a circular orifice or a ribbon orifice. Test subjects were asked to either vertically or horizontally apply the emulsion composition directly to their upper teeth. The test subjects were asked to do two successive applications for a 15 second period each applying the emulsion composition to the top 6 front teeth covering central incisors, lateral incisors and canines. The evenness of each applicator device was assessed subjectively with 8 or more graders who evaluated images before and after application and graded on a scale of 0-5 with five being very even application.

The percent of emulsion composition transferred from applicators FIG. 2A and FIG. 3A to the tooth surface was evaluated. Approximately 0.075 g of the emulsion composition was loaded onto each described applicator device. The percent emulsion composition transferred was evaluated by comparing the displaced weight of the applicator devices pre- and post-application. As shown in TABLE 1, the applicator device of FIG. 2A illustrated higher transfer of the emulsion complex onto the upper front teeth surfaces.

**TABLE 1. Percent Emulsion composition transferred with Composition applied with Circular Orifice**

| Applicator | Horizontally Applied (SD) |
|---|---|
| FIG. 2A | 74.30 (4.45) |
| FIG. 3A | 64.35 (4.45) |

Approximately 0.075 g of the emulsion composition was loaded onto the applicator devices of FIG. 2A and FIG 3A and test subjects were asked to either vertically or horizontally apply the emulsion composition directly to their upper teeth. The test subjects were asked to do two successive applications for a 15 second period each applying the emulsion composition to the top 6 front teeth covering central incisors, lateral incisors and canines. The evenness of each applicator device was assessed subjectively with 8 or more graders who evaluated images before and after application and graded on a scale of 0-5 with five being very even application. The applicator device FIG 2A demonstrated more even applicator of the emulsion composition to the tooth surface regardless of application orientation

**TABLE 2. Evenness of Application**

| Applicator | Horizontally Applied (SD) | Vertically Applied (SD) |
|---|---|---|
| FIG. 2A | 2.66 (0.17) | 2.53 (0.24) |
| FIG. 3A | 2.30 (0.17) | 2.26 (0.24) |

**TABLE 3. Evenness of application both vertical and horizontal combined**

| Applicator | Evenness (SD) |
|---|---|
| FIG. 2A | 2.60 (0.14) |
| FIG. 3A | 2.28 (0.14) |

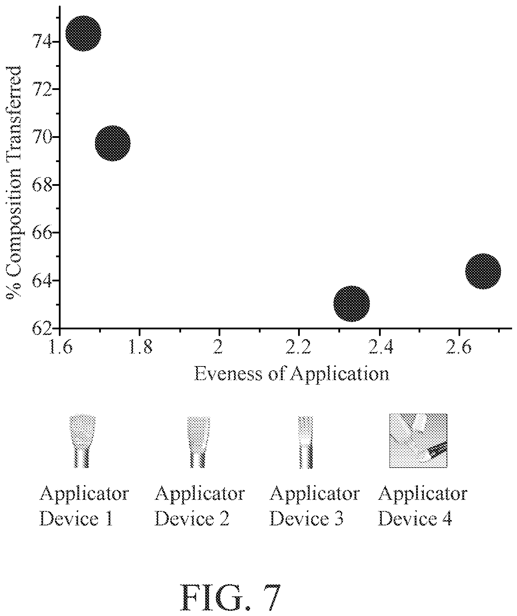

FIG. 7 displays the % composition transferred relative to the evenness of application of the composition from four different applicator devices. Applicator Device 1 was the applicator device of FIG. 1A. Applicator Device 4 was the applicator device of FIG. 3A. Applicator Devices 2-3 were additional inventive applicator devices.

As shown in FIG. 7, Applicator Device 1 had the highest evenness of application of all devices tested, but a lower amount of % composition transferred than Applicator Device 4, a comparative applicator device. Thus, it was investigated whether the % composition transferred of Applicator Device 1 could be further improved by modifying the applicator tip and/or the dispenser nozzle.

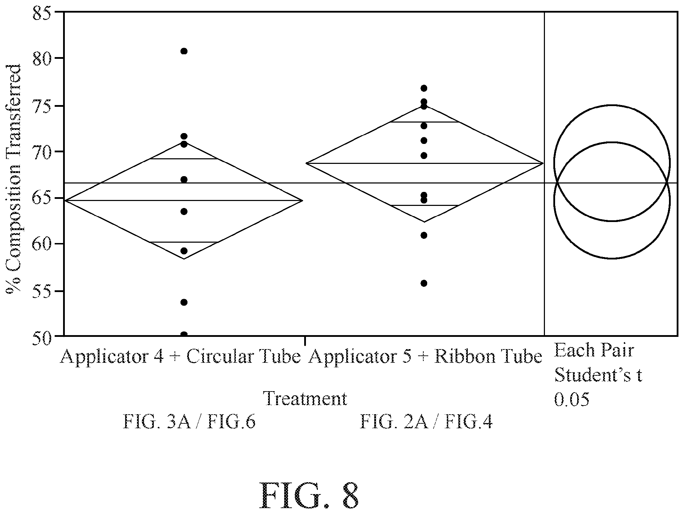

As shown in FIG. 8, Applicator Device 5, the applicator device of FIG. 2A, was paired with a container with a ribbon orifice in the nozzle, as shown in FIG. 4. This kit was directly compared to Applicator Device 4, the applicator device of FIG. 3A paired with a container with a circular orifice in the nozzle as shown in FIG. 6. TABLE 4 shows that the pair of Applicator Device 5 with a ribbon orifice will lead to a higher % emulsion composition transferred to the surface of teeth.

**TABLE 4. Percent Emulsion composition transferred**

| Applicator | Tube Orifice | Horizontally Applied (SD) | Vertically Applied (SD) | Combined Applied (SD) |
|---|---|---|---|---|
| FIG. 2A | FIG. 5 | 74.14 (2.18) | 63.25 (2.18) | 68.10 (2.18) |
| FIG. 3A | FIG. 6 | 72.17 (3.02) | 57.20 (3.02) | 64.60 (3.02) |

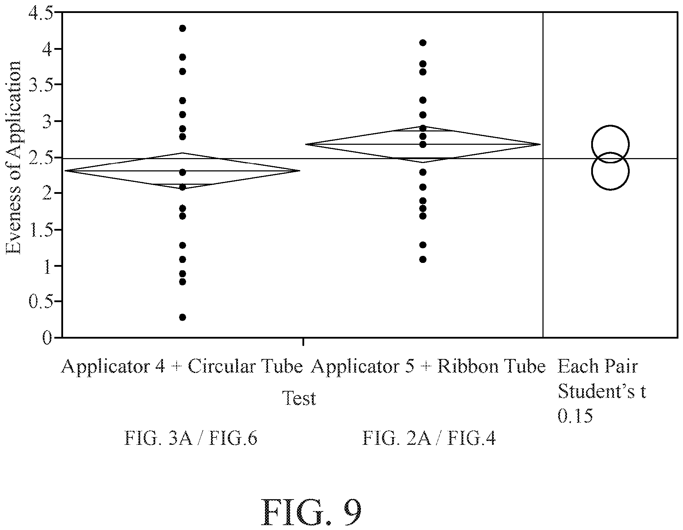

FIG. 9 shows that the evenness of application will remain higher with Applicator Device 5 compared with Applicator Device 4.

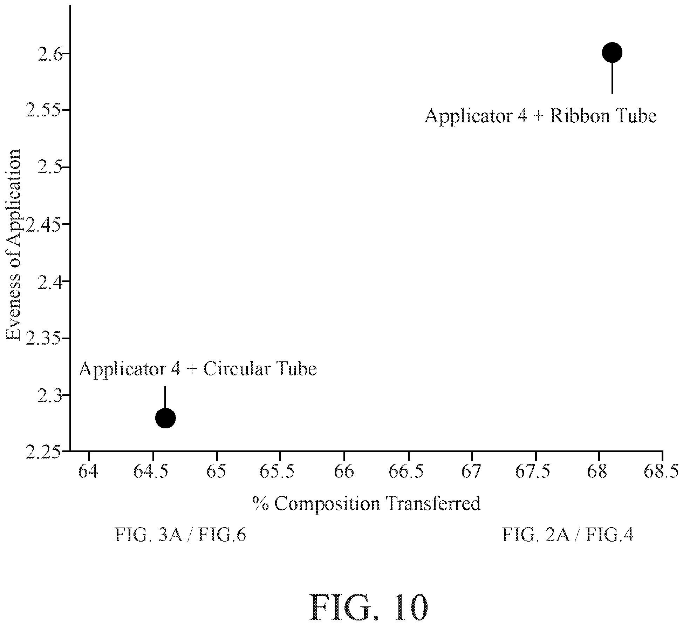

FIG. 10 displays the % composition transferred and the evenness of application of Applicator Device 5 dispensed from a ribbon orifice will be higher than the use of Applicator Device 4 dispensed from a circular orifice.

In the *in vitro* model, the emulsion composition was loaded onto various applicator devices from a container with a nozzle having either a circular orifice or a ribbon orifice. The applicator device was then swiped once onto a polystyrene surface coated with saliva to contact the applicator device to the surface of the polystyrene, which was determined to have a water contact angle of between 20 and 30 degrees. In comparison, the water contact angle of a tooth's surface in the oral cavity of a test subject was determined to be approximately 30-40 degrees.

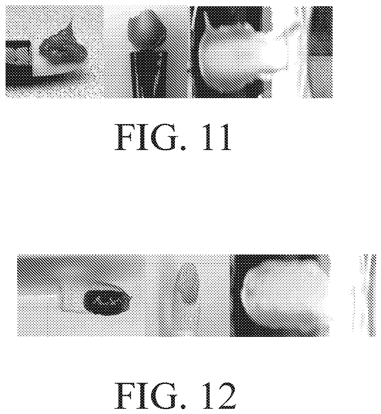

As shown in FIG. 12, application of a composition with the applicator device of FIG. 3A when dispensed from a circular orifice left a substantial amount of emulsion on the applicator device after a single swipe. The comparative applicator also led to an uneven application with areas of higher application and area of lower application.

In contrast, as shown in FIG. 11, application of a composition with the applicator device of FIG. 2A when dispensed from a ribbon orifice left only a minimal amount of emulsion on the applicator device after a single swipe. Additionally, the applicator device of FIG. 2A resulting in a more consistent application of the emulsion composition.

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention.

## Claims

1. A kit for applying an oral care composition to an oral cavity surface comprising:
(a) an applicator device (10), the applicator device (10) comprising an applicator handle (20) suitable for self-application and an applicator tip (30) and:
(i) at least one external ridge (31), wherein the at least one external ridge (31) has a first height and lies along at least a majority of the perimeter of the applicator tip (30) such that the at least one external ridge (31) forms an internal cavity (35) for the emulsion composition to be placed in; and
(ii) a plurality of internal ridges (32), wherein the plurality of internal ridges (32) has a second height and extends in a direction at least substantially parallel or parallel to the longitudinal axis of the applicator device (10);
wherein the first height is greater than the second height and wherein the internal cavity (35) is formed from the difference in heights of the at least one external ridge (31) and the plurality of internal ridges (32); and
(iii) a plurality of internal channels (33) between the plurality of internal ridges (32);
and
(b) a container (50) for the oral care composition, the container (50) comprising:
(i) a nozzle (51) comprising a ribbon orifice (52), and
(ii) a storage portion (54).

2. The kit of claim 1, wherein the applicator tip (30) comprises a durometer rating of from about 45 A to about 75 A, or preferably from about 60 A to about 70 A.

3. The kit of any one of claims 1 or 2, wherein the plurality of internal ridges (32) comprises from 2 to about 20 internal ridges (32).

4. The kit of any one of claims 1 to 3, wherein the applicator tip (30) is made from an elastomer, preferably wherein the elastomer is a thermoplastic elastomer, a styrenic, a copolyester, a polyurethane, a polyamide, a polyolefin blend, a polyolefin alloy, a reactor TPO, a polyolefin plastomer, a polyolefin elastomer, and/or combinations thereof.

5. The kit of any one of claims 1 to 4, wherein an oral care composition is comprised in the storage portion (54), wherein the oral care composition comprises an emulsion composition with a cone penetration value of from about 100 to about 300.

6. The kit of claim 1, wherein the internal cavity (35) has a width comprising a length (W3) and the ribbon orifice (52) has a width comprising the length (W4), wherein the length (W4) of the width of the ribbon orifice (52) is equal to or less than the length (W3) of the width of the internal cavity (35).

7. The kit of claim 6, wherein the length (W4) of the ribbon orifice (52) is from about 5% less than to about 40% less than about the length (W3) of the internal cavity (35), preferably wherein the length (W4) of the ribbon orifice (52) is from about 1% less than to about 10% less than the length (W3) the internal cavity (35).

8. The kit of claim 6 or 7, wherein the storage portion (54) comprises an oral care composition, wherein the % composition transferred from the applicator device (10) to the oral cavity surface is at least about 65%.

9. The kit of any one of claims 6 to 8, wherein the evenness of application is at least about 2.4.

10. The kit of any one of claims 6 to 9, wherein the second distance is from about 1.0 mm to about 10 mm.

## Patentansprüche

1. Kit zum Applizieren einer Mundpflegezusammensetzung auf einer Mundhöhlenoberfläche, umfassend:
(a) eine Applikatorvorrichtung (10), die Applikatorvorrichtung (10) umfassend einen Applikatorgriff (20), der für eine Selbstapplikation geeignet ist, und eine Applikatorspitze (30); und:
(i) wenigstens einen externen Kamm (31), wobei der wenigstens eine externe Kamm (31) eine erste Höhe aufweist und entlang wenigstens eines Großteils des äußeren Rands der Applikatorspitze (30) derart liegt, dass der wenigstens eine externe Kamm (31) einen inneren Hohlraum (35) ausbildet, in den die Emulsionszusammensetzung platziert werden soll; und
(ii) eine Vielzahl von inneren Kämmen (32), wobei die Vielzahl von inneren Kämmen (32) eine zweite Höhe aufweist und sich in einer Richtung wenigstens im Wesentlichen parallel oder parallel zu der Längsachse der Applikatorvorrichtung (10) erstreckt;
wobei die erste Höhe größer als die zweite Höhe ist und wobei der innere Hohlraum (35) aus den Höhenunterschieden des wenigstens einen externen Kamms (31) und der Vielzahl von inneren Kämmen (32) ausgebildet ist; und
(iii) eine Vielzahl von inneren Kanälen (33) zwischen der Vielzahl von inneren Kämmen (32); und
(b) einen Behälter (50) für die Mundpflegezusammensetzung, der Behälter (50) umfassend:
(i) eine Düse (51), umfassend eine Bandöffnung (52), und
(ii) einen Speicherabschnitt (54).

2. Kit nach Anspruch 1, wobei die Applikatorspitze (30) eine Durometerbewertung von etwa 45 A bis etwa 75 A oder vorzugsweise von etwa 60 A bis etwa 70 A umfasst.

3. Kit nach einem der Ansprüche 1 oder 2, wobei die Vielzahl von inneren Kämmen (32) von 2 bis etwa 20 innere Kämme (32) umfasst.

4. Kit nach einem der Ansprüche 1 bis 3, wobei die Applikatorspitze (30) aus einem Elastomer hergestellt ist, vorzugsweise wobei das Elastomer ein Thermoplastelastomer, ein Styrol, ein Copolyester, ein Polyurethan, ein Polyamid, ein Polyolefinblend, eine Polyolefinlegierung, ein Reaktor-TPO, ein Polyolefin-Plastomer, ein Polyolefinelastomer und/oder Kombinationen davon ist.

5. Kit nach einem der Ansprüche 1 bis 4, wobei eine Mundpflegezusammensetzung in dem Speicherabschnitt (54) enthalten ist, wobei die Mundpflegezusammensetzung eine Emulsionszusammensetzung mit einem Konuspenetrationswert von etwa 100 bis etwa 300 umfasst.

6. Kit nach Anspruch 1, wobei der innere Hohlraum (35) eine Breite aufweist, umfassend eine Länge (W3), und die Bandöffnung (52) eine Breite aufweist, umfassend die Länge (W4), wobei die Länge (W4) der Breite der Bandöffnung (52) gleich oder kleiner als die Länge (W3) der Breite des inneren Hohlraums (35) ist.

7. Kit nach Anspruch 6, wobei die Länge (W4) der Bandöffnung (52) von etwa 5 % kleiner als bis etwa 40 % kleiner als etwa die Länge (W3) des inneren Hohlraums (35) ist, vorzugsweise wobei die Länge (W4) der Bandöffnung (52) von etwa 1 % kleiner als bis etwa 10 % kleiner als die Länge (W3) des inneren Hohlraums (35) ist.

8. Kit nach Anspruch 6 oder 7, wobei der Speicherabschnitt (54) eine Mundpflegezusammensetzung umfasst, wobei die %-Zusammensetzung, die von der Applikatorvorrichtung (10) auf die Mundhöhlenoberfläche überführt wird, wenigstens etwa 65 % ist.

9. Kit nach einem der Ansprüche 6 bis 8, wobei die Gleichmäßigkeit einer Applikation wenigstens etwa 2,4 ist.

10. Kit nach einem der Ansprüche 6 bis 9, wobei der zweite Abstand von etwa 1,0 mm bis etwa 10 mm ist.

## Revendications

1. Trousse permettant d'appliquer une composition de soins bucco-dentaires à une surface de cavité buccale comprenant :
(a) un dispositif applicateur (10), le dispositif applicateur (10) comprenant une poignée d'applicateur (20) appropriée pour une auto-application et un embout applicateur (30) et :
(i) au moins une crête externe (31), dans laquelle l'au moins une crête externe (31) a une première hauteur et repose le long d'au moins une majorité du périmètre de la pointe d'applicateur (30) de telle sorte que l'au moins une crête externe (31) forme une cavité interne (35) pour placer la composition d'émulsion ; et
(ii) une pluralité de crêtes internes (32), dans laquelle la pluralité de crêtes internes (32) a une seconde hauteur et s'étend dans une direction au moins sensiblement parallèle ou parallèle à l'axe longitudinal du dispositif applicateur (10) ;
dans laquelle la première hauteur est supérieure à la seconde hauteur et dans laquelle la cavité interne (35) est formée à partir de la différence de hauteurs de l'au moins une crête externe (31) et de la pluralité de crêtes internes (32) ; et
(iii) une pluralité de canaux internes (33) entre la pluralité de crêtes internes (32) ; et
(b) un récipient (50) pour la composition de soins bucco-dentaires, le récipient (50) comprenant :
(i) une buse (51) comprenant un orifice de ruban (52), et
(ii) une partie de stockage (54).

2. Trousse selon la revendication 1, dans laquelle la pointe applicatrice (30) comprend un indice duromètre d'environ 45 A à environ 75 A, ou de préférence d'environ 60 A à environ 70 A.

3. Trousse selon l'une quelconque des revendications 1 ou 2, dans laquelle la pluralité de crêtes internes (32) comprend de 2 à environ 20 crêtes internes (32).

4. Trousse selon l'une quelconque des revendications 1 à 3, dans laquelle l'embout pour applicateur (30) est fabriqué à partir d'un élastomère, de préférence dans laquelle l'élastomère est un élastomère thermoplastique, un styrénique, un copolyester, un polyuréthane, un polyamide, un mélange de polyoléfines, un alliage polyoléfinique, un TPO issu d'un réacteur un plastomère de polyoléfine, un élastomère polyoléfinique, et/ou des combinaisons de ceux-ci.

5. Trousse selon l'une quelconque des revendications 1 à 4, dans laquelle une composition de soins bucco-dentaires est comprise dans la partie de stockage (54), dans laquelle la composition de soins bucco-dentaires comprend une composition d'émulsion avec une valeur de pénétration de cône allant d'environ 100 à environ 300.

6. Trousse selon la revendication 1, dans laquelle la cavité interne (35) a une largeur comprenant une longueur (W3) et l'orifice de ruban (52) a une largeur comprenant la longueur (W4), dans laquelle la longueur (W4) de la largeur de l'orifice de ruban (52) est égale ou inférieure à la longueur (W3) de la largeur de la cavité interne (35).

7. Trousse selon la revendication 6, dans laquelle la longueur (W4) de l'orifice de ruban (52) est d'environ 5 % inférieure à environ 40 % inférieure à environ la longueur (W3) de la cavité interne (35), de préférence dans laquelle la longueur (W4) de l'orifice de ruban (52) est d'environ 1 % inférieure à environ 10 % inférieure à la longueur (W3) de la cavité interne (35).

8. Trousse selon la revendication 6 ou 7, dans laquelle la partie de stockage (54) comprend une composition de soins bucco-dentaires, dans laquelle le pourcentage de la composition transférée depuis le dispositif applicateur (10) vers la surface de cavité buccale est d'au moins environ 65 %.

9. Trousse selon l'une quelconque des revendications 6 à 8, dans laquelle l'uniformité de l'application est d'au moins 2,4.

10. Trousse selon l'une quelconque des revendications 6 à 9, dans laquelle la seconde distance est d'environ 1,0 mm à environ 10 mm.
